# EUROPEAN PATENT APPLICATION

(11) **EP 4 094 727 A1**
(43) Date of publication of application: **30.11.2022**
(21) Application number: 21744854.7
(22) Date of filing: 15.01.2021
(51) Int. Cl.: A61F 2/34

(54) **ACETABULAR CUP PROSTHESIS AND LOCKING STRUCTURE THEREFOR**

(30) Priority: 21.01.2020 CN 202020138816 U; 21.01.2020 CN 202010071018
(71) Applicant: Beijing Naton Medical Technology Holdings Co., Ltd., Beijing 100094 (CN); Bricon GmbH, 78573 Wurmlingen (DE)
(72) Inventor: HU, Xiaoqiang, Beijing 100094 (CN); DONG, Xiang, Beijing 100094 (CN)
(74) Representative: Ege Lee & Roider Patentanwälte
(86) International application number: PCT/CN2021/072291
(87) International publication number: WO 2021/147789

(57) **Abstract**

Disclosed are an acetabular cup prosthesis and a locking structure therefor. The acetabular cup prosthesis comprises an outer cup (1) and a liner (2), in which an inner surface of the outer cup (1) is provided with an abutting face (11) in a circumferential direction thereof; an outer surface of the liner (2) is provided with a protruding snap ring (21) at a bearing part, and is provided with an elastic locking tongue (22) at a non-bearing part; and the protruding snap ring (21) and the elastic locking tongue (22) both abut against the abutting face (11), such that the liner (2) is self-locked in the outer cup (1). The acetabular cup prosthesis can ensure the thickness of the liner (2) to the maximum possible extent and can minimize the volume of bone-cut for a patient, and is easy to load, is not prone becoming disengaged, and is firmly fixed.

## Description

### CROSS-REFERENCE TO RELATED APPLICATIONS

This application claims priority to and benefit to the Chinese Patent Application No. 202020138816.1 and the Chinese Patent Application No. 202010071018.6, both filed on January 21, 2020, the entire contents of which are incorporated herein by reference.

### TECHNICAL FIELD

Embodiments of the present disclosure relate to the field of medical prosthesis technologies, and more particularly, to an acetabular cup prosthesis and a locking structure thereof.

### BACKGROUND

An acetabular cup prosthesis is one of the widely used prostheses in the hip replacement. A structure of the acetabular cup prosthesis is generally composed of a metal outer cup and a polyethylene liner. During the operation, the metal outer cup is first driven into the human acetabulum, and then the polyethylene liner is mounted into the metal outer cup. On the one hand, it is necessary for doctors to easily mount the polyethylene liner into the metal outer cup during surgery; on the other hand, because the acetabular cup prosthesis has to bear continuous impacts from human weight and walking load for many years after mounted, the polyethylene liner is required to be closely fitted and stably fixed in the metal outer cup without loosening and coming out. Therefore, the assembly of the liner in the outer cup should achieve three main functions: it is easy to mount, not easy to come out, and firmly fixed.

Since the outer cup and the liner are in contact with each other through an inner spherical surface and an outer spherical surface, in order to achieve a perfect close fit, the two spherical surfaces are required to be completely consistent, which is almost impossible due to the existence of errors in the manufacturing process. In addition, a locking structure of the outer cup and the liner shall meet the requirements that the liner is easy to drive in while being firmly locked and hard to come out, which is a contradiction. Therefore, many existing acetabular products are either firmly locked but hard to drive in; or, it is easy to drive in, but there is a problem of easy loosening.

In addition, in order to ensure the sufficient strength and the wear resistance, the polyethylene liner is required to reach a certain thickness at a main bearing portion, generally not less than 5mm, and the thicker the better. In order to minimize the volume of bone-cut for a patient, the implanted acetabular cup prosthesis should not be too large. Therefore, the polyethylene liner should not be too thick. However, since the liner with an original limited thickness has to be provided with the required locking structure, it is difficult to guarantee a minimum thickness of the bearing portion.

### SUMMARY

To this end, an embodiment of the present disclosure proposes an acetabular cup prosthesis, which can guarantee a thickness of the liner to a maximum extent, minimize the volume of bone-cut for a patient, and is easy to mount, not easy to come out, and firmly fixed.

Another embodiment of the present disclosure proposes a locking structure for an acetabular cup prosthesis, which can guarantee a thickness of the liner to a maximum extent, minimize the volume of bone-cut for a patient, and is easy to mount, not easy to come out, and firmly fixed.

The acetabular cup prosthesis according to embodiments of a first aspect of the present disclosure includes an outer cup and a liner. An inner surface of the outer cup is provided with an abutting face along a circumferential direction. An outer surface of the liner is provided with a protruding ring at a bearing portion and an elastic locking tongue at a non-bearing portion. The protruding ring and the elastic locking tongue each abut against the abutting face to self-lock the liner in the outer cup.

In the acetabular cup prosthesis according to embodiments of the present disclosure, the liner is an asymmetric locking structure, and in this design, the locking structure is divided into two portions. The elastic locking tongue and other structures that need to lose a thickness of the liner are placed at the non-bearing portion, so that an excessive size of the acetabular cup prosthesis caused by a too thick liner is avoided, thus minimizing the volume of bone-cut for a patient. The bearing portion adopts the design of the fixed protruding ring, to guarantee the thickness of the liner to a maximum extent and to ensure that the liner has sufficient strength and wear resistance. The structure of the bearing portion and the structure of the non-bearing portion of the liner are jointly fitted with the abutting face on the outer cup to lock the liner. Moreover, when the liner is locked in the outer cup, the elastic locking tongue is pressed on the abutting face of the outer cup by an elastic force, and forms an outward supporting elastic structure together with the spherical surface under the liner, to eliminate the possible gap due to manufacturing errors, and to tightly press the outer spherical surface of the liner on the inner spherical surface of the outer cup. Therefore, the acetabular cup prosthesis of the present disclosure can guarantee the thickness of the liner to a maximum extent, minimize the volume of bone-cut for a patient, and is easy to mount, not easy to come out, and firmly fixed.

In the locking structure for the acetabular cup prosthesis according to embodiments of a second aspect of the present disclosure, the acetabular cup prosthesis includes an outer cup and a liner, and the liner is locked in the outer cup through the locking structure. An inner surface of the outer cup is provided with an abutting face along a circumferential direction. An outer surface of the liner is provided with a protruding ring at a bearing portion and an elastic locking tongue at a non-bearing portion. The protruding ring and the elastic locking tongue each abut against the abutting face to self-lock the liner in the outer cup.

In the locking structure of the acetabular cup prosthesis according to embodiments of the present disclosure, the liner is an asymmetric locking structure, and in this design, the locking structure is divided into two portions. The elastic locking tongue and other structures that need to lose a thickness of the liner are placed at the non-bearing portion, so that an excessive size of the acetabular cup prosthesis caused by a too thick liner is avoided, thus minimizing the volume of bone-cut for a patient. The bearing portion adopts the design of the fixed protruding ring, to guarantee the thickness of the liner to a maximum extent and to ensure that the liner has sufficient strength and wear resistance. The structure of the bearing portion and the structure of the non-bearing portion of the liner are jointly fitted with the abutting face on the outer cup to lock the liner. Moreover, when the liner is locked in the outer cup, the elastic locking tongue is pressed on the abutting face of the outer cup by an elastic force, and forms an outward supporting elastic structure together with the spherical surface under the liner, to eliminate the possible gap due to manufacturing errors, and to tightly press the outer spherical surface of the liner on the inner spherical surface of the outer cup. Therefore, the locking structure of the acetabular cup prosthesis of the present disclosure can guarantee the thickness of the liner to a maximum extent, minimize the volume of bone-cut for a patient, and is easy to mount, not easy to come out, and firmly fixed.

In some embodiments, the inner surface of the outer cup defines an annular groove with a triangular longitudinal section along the circumferential direction, and a side wall of the annular groove close to an opening portion of the outer cup is the abutting face; or, the inner surface of the outer cup is provided with an annular protrusion along the circumferential direction, and a side wall of the annular protrusion away from the opening portion of the outer cup is the abutting face.

In some embodiments, the elastic locking tongue is formed by digging a tangential groove in the outer surface of the liner, and an orientation of the elastic locking tongue is facing away from a bottom of the liner.

In some embodiments, an extension line of a force of the abutting face on the elastic locking tongue is located below a midpoint at a root of the elastic locking tongue.

In some embodiments, the elastic locking tongue has an inner side face, an outer side face parallel to the inner side face, and a top face perpendicular to the inner side face and the outer side face, an angle between the abutting face and the top face of the elastic locking tongue is 0-5 degrees.

In some embodiments, an angle between the outer side face of the elastic locking tongue and a central axis of the liner is 40-50 degrees.

In some embodiments, an angle occupied by the protruding ring in the circumferential direction is 90-180 degrees.

In some embodiments, the protruding ring is a continuous unitary section or a plurality of sections spaced apart from each other.

In some embodiments, an outer edge of an opening of the liner is provided with a protrusion, and an inner edge of an opening of the outer cup is provided with a recessed portion matching the protrusion.

In some embodiments, a connection line is formed between any point on the abutting face and a center of a circle of the opening portion of the outer cup, and an angle between the connection line and a horizontal plane is greater than or equal to 45 degrees.

### BRIEF DESCRIPTION OF THE DRAWINGS

FIG. 1 is a schematic view of an outer cup of an acetabular cup prosthesis according to the present disclosure, in which (a) is a perspective view, (b) is a top view, and (c) is a sectional view.
FIG. 2 is a schematic view of a liner of an acetabular cup prosthesis according to the present disclosure, in which (a) is a perspective view, (b) is a top view, and (c) is a sectional view.
FIG. 3 is a schematic view of an acetabular cup prosthesis according to the present disclosure in a combined state, in which (a) is a perspective view and (b) is a sectional view.
FIG. 4 is a schematic view of an acetabular cup prosthesis according to the present disclosure in a use state.
FIG. 5 is a schematic view of a force direction of an outer cup on a liner of the acetabular cup prosthesis according to the present disclosure.
FIG. 6 is a schematic view of an angle of a contact portion of an outer cup and a liner of an acetabular cup prosthesis according to the present disclosure.

### DETAILED DESCRIPTION

Embodiments of the present disclosure are described in detail below, and examples of the embodiments are illustrated in accompanying drawings. The following embodiments described with reference to the accompanying drawings are exemplary and are only intended to explain the present disclosure, rather than limit the present disclosure. In the description of the present disclosure, it shall be understood that terms such as "central," "longitudinal," "transverse," "length," "width," "thickness," "upper," "lower," "front," "rear," "left," "right," "vertical," "horizontal," "top," "bottom," "inner," "outer," "clockwise," "counterclockwise," "axial," "radial" and "circumferential" should be construed to refer to the orientation and position as then described or as illustrated in the drawings under discussion. These relative terms are only for convenience of description and do not indicate or imply that the device or element referred to must have a particular orientation, or be constructed and operated in a particular orientation. Thus, these terms shall not be construed as limitation on the present disclosure.

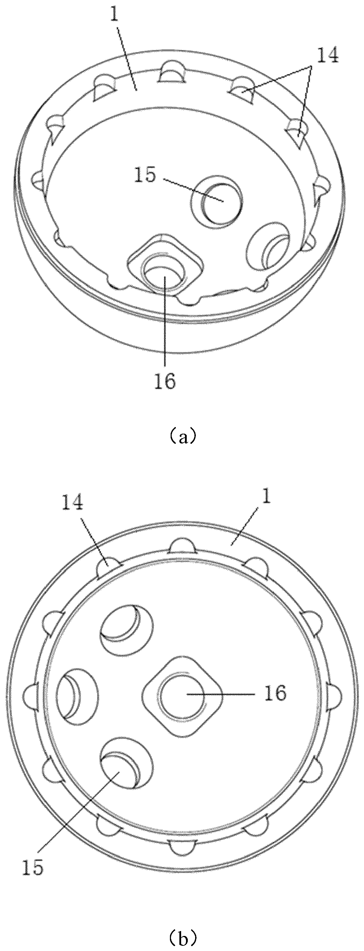

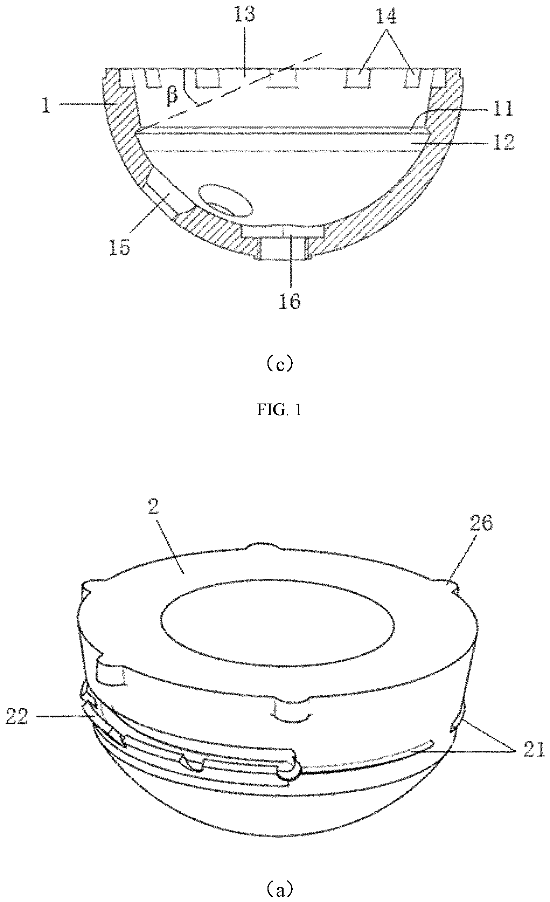

As illustrated in FIGS. 1 to 6, an acetabular cup prosthesis according to embodiments of the present disclosure includes an outer cup 1 and a liner 2. An inner surface of the outer cup 1 is provided with an abutting face 11 along a circumferential direction. Specifically, the abutting face 11 may be realized in the following several modes:
Mode 1:
   As illustrated in FIG. 1, an annular groove 12 with a triangular longitudinal section is arranged in the inner surface of the outer cup 1 along the circumferential direction, and a side wall of the annular groove 12 close to an opening portion 13 of the outer cup is the abutting face 11.
Mode 2:
   An annular protrusion (not shown) is arranged on the inner surface of the outer cup along the circumferential direction, and a side wall of the annular protrusion away from the opening portion of the outer cup is the abutting face.
Mode 3:
   It is conceivable for those skilled in the art that the abutting face may also be formed by the above mode 1 and mode 2 together.

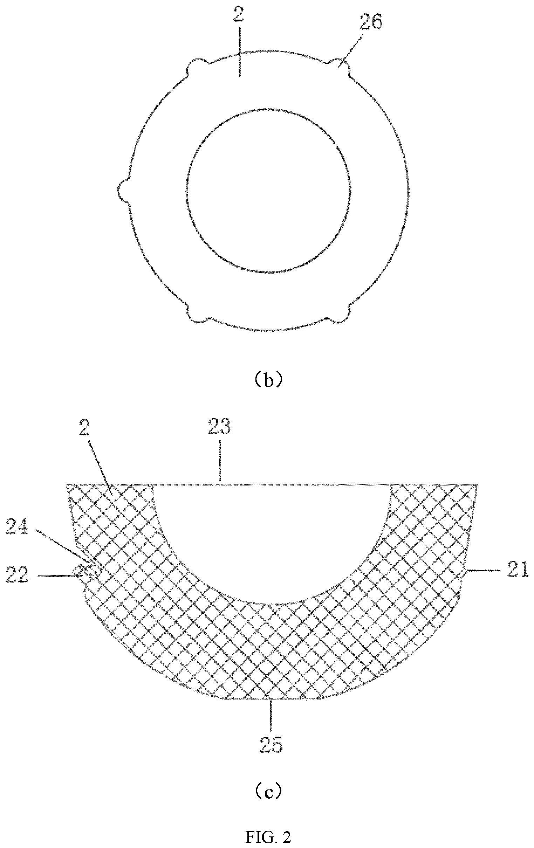

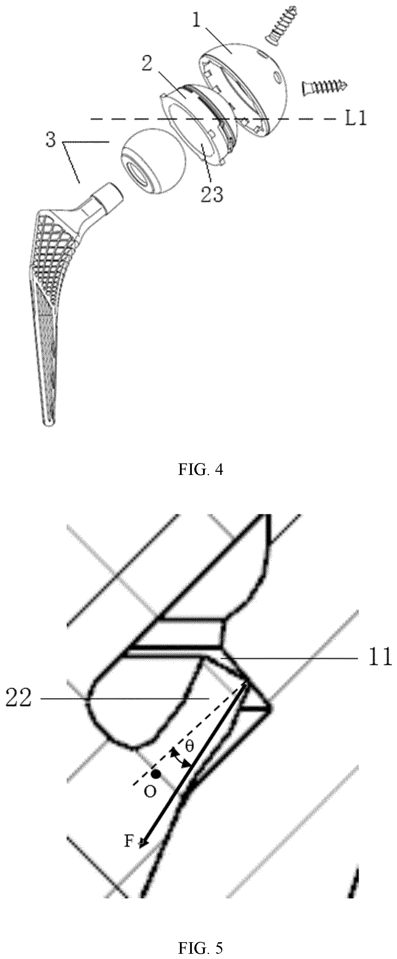

As illustrated in FIG. 2, a protruding ring 21 is arranged on an outer surface of the liner 2 at a bearing portion, and an elastic locking tongue 22 is arranged on the outer surface of the liner 2 at a non-bearing portion. The bearing portion refers to a portion which plays a main bearing role after the acetabular cup prosthesis is implanted into the human body, and the non-bearing portion refers to a portion which plays a non-main bearing role after the acetabular cup prosthesis is implanted into the human body. It is well known that after the acetabular cup prosthesis is implanted into the human body, the liner is oriented so that an opening portion 23 is inclined downward, and an inner side of the liner is fitted with a femoral head prosthesis 3. As illustrated in FIG. 4, a portion of the liner 2 above a horizontal line L1 is the bearing portion, and a portion of the liner 2 below the horizontal line L1 is the non-bearing portion. The bearing portion usually accounts for about 1/3 of an overall area of the liner 2.

In the bearing portion, a design of the fixed protruding ring 21 is adopted, which does not lead to lose a thickness of the liner due to the snap structure, and may guarantee the thickness of the liner 2 to a maximum extent. In the non-bearing portion, a design of the elastic locking tongue 22 is adopted, which may obtain the elasticity to a maximum extent, and facilitate the implantation of the liner 2 while ensuring the fixation effect of the liner 2 and the outer cup 1. The elastic locking tongue 22 is preferably formed by digging a tangential groove 24 in the outer surface of the liner 2, and the tangential groove 24 consumes a certain thickness of the liner 2, thus reducing the volume of bone-cut for a patient to a certain extent. An orientation of the elastic locking tongue 22 is facing away from a bottom 25 of the liner 2.

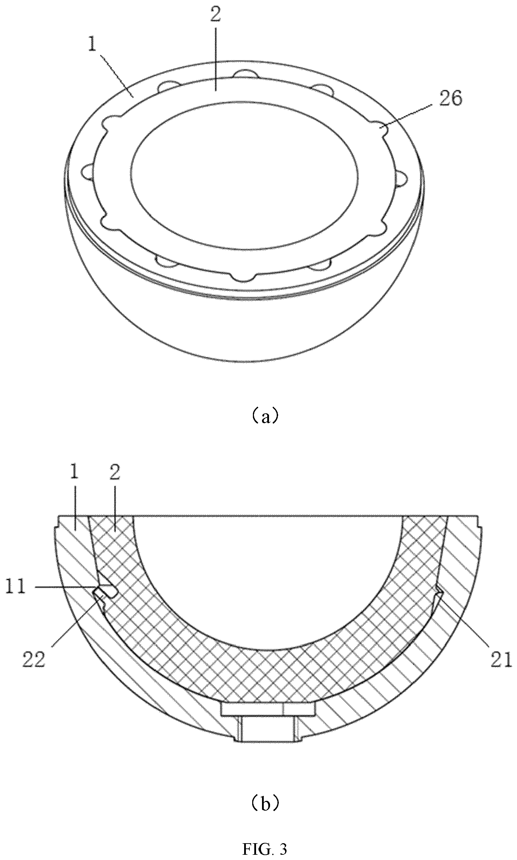

When in use, the protruding ring 21 and the elastic locking tongue 22 on the liner 2 abut against the abutting face 11 on the outer cup 1, to self-lock the liner 2 in the outer cup 1. Specifically, when the liner 2 is pressed into the outer cup 1, the elastic locking tongue 22 of the liner 2 may be compressed inward to facilitate assembly. After mounted, the elastic locking tongue 22 pops out by the elasticity, and is supported against the abutting face 11 of the outer cup 1, so that an outer spherical surface of the liner 2 and an inner spherical surface of the outer cup 1 are tightly pressed against each other (FIG. 3), thus forming a self-locking. An extension/height of the abutting face 11 in a vertical direction compensates for the manufacturing errors existing in the outer cup 1 and the liner 2. As long as the manufacturing errors of the two components are within a required tolerance range, the elastic locking tongue 22 of the liner 2 may be supported against the abutting face 11 of the outer cup 1 to tightly press fit and fix spherical surfaces of the two components without a gap.

In the present disclosure, the liner is an asymmetric locking structure, and in this design, the locking structure is divided into two portions. The elastic locking tongue and other structures that need to lose the thickness of the liner are placed at the non-bearing portion, so that an excessive size of the acetabular cup prosthesis caused by a too thick liner is avoided, thus minimizing the volume of bone-cut for a patient. The bearing portion adopts the design of the fixed protruding ring, to guarantee the thickness of the liner to a maximum extent and to ensure that the liner has sufficient strength and wear resistance. The structure of the bearing portion and the structure of the non-bearing portion of the liner are jointly fitted with the abutting face on the outer cup to lock the liner. Moreover, when the liner is locked in the outer cup, the elastic locking tongue is pressed on the abutting face of the outer cup by an elastic force, and forms an outward supporting elastic structure together with the spherical surface under the liner, to eliminate the possible gap due to manufacturing errors, and to tightly press the outer spherical surface of the liner on the inner spherical surface of the outer cup. Therefore, the acetabular cup prosthesis of the present disclosure can guarantee the thickness of the liner to a maximum extent, minimize the volume of bone-cut for a patient, and is easy to mount, not easy to come out, and firmly fixed.

As illustrated in FIGS. 5 to 6, when the liner 2 of the acetabular cup prosthesis receives an outward thrust, an external force F acting on a top of the elastic locking tongue 22 may be decomposed into a normal pressure perpendicular to the abutting face 11 and a friction force between the elastic locking tongue 22 and the abutting face 11 parallel to the abutting face 11. θ is a friction angle between the elastic locking tongue 22 and the abutting face 11. For example, the friction angle θ between the polyethylene and the titanium alloy is about 10-15 degrees. In order to ensure that the liner 2 is not easy to come out, an extension line of the force F of the abutting face 11 on the elastic locking tongue 22 is preferably located below a midpoint O at a root of the elastic locking tongue 22, so that the elastic locking tongue 22 always presses towards a bottom of the abutting face 11 of the outer cup when deformed under the external force, so as to lock the liner 2 and make it not easy to come out. To further achieve this purpose, the elastic locking tongue 22 preferably has an inner side face 221, an outer side face 222 parallel to the inner side face 221, and a top face 223 perpendicular to both the inner side face 221 and the outer side face 222. An angle α between the abutting face 11 and the top face 223 of the elastic locking tongue 22 is 0-5 degrees, so that a contact point of the two surfaces is kept at an outer cuspidal point on a top of the elastic locking tongue 22 (i.e., an intersection point of the outer side face 222 and the top face 223), resulting in an extension line of a resultant force of the normal pressure and the friction force further moving downward to enhance the locking function. The O point may be a center point of a root connection line of the elastic locking tongue 22. Specifically, the root connection line is a straight line formed by a connection point of the inner side face 221 and the liner 2 and a connection point of the outer side face 222 and the liner 2 connected to each other.

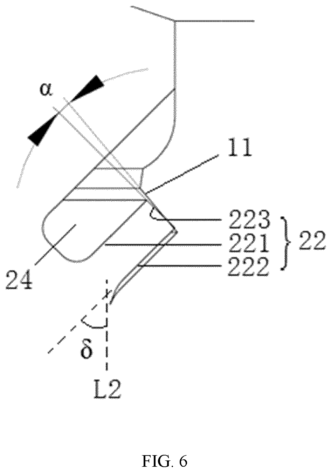

As illustrated in FIG. 6, in order to minimize a resistance during mounting of the liner 2, an angle δ between the elastic locking tongue 22 and the vertical direction (i.e., an angle between the outer side face 222 (or its extension line) of the elastic locking tongue 22 and a central axis of the liner 2 (the vertical line L2 as illustrated in the figure) shall be as small as possible to reduce the resistance, but a too small angle δ reduces an outward popping range of the elastic locking tongue 22 and affects the locking of the liner 2. A preferred value is 40-50 degrees.

In embodiments of the present disclosure, as illustrated in FIG. 2, an angle occupied by the protruding ring 21 in the circumferential direction is preferably 90-180 degrees, and the protruding ring 21 works together with the opposite elastic locking tongue 22 to lock the liner 2. If the angle is too small, the bearing portion/range of the acetabular cup prosthesis is not sufficiently covered. If the angle exceeds 180 degrees, the interference caused by the lack of elasticity of the protruding ring 21 occurs, while the reliability of the fixation of the liner 2 is reduced. In addition, the protruding ring 21 may be a continuous unitary section or a plurality of sections spaced apart from each other. A height of the protruding ring 21 beyond the outer surface of the liner 2 may be different from a height of the elastic locking tongue 22 beyond the outer surface of the liner 2. Preferably, the height of the protruding ring 21 is lower than the height of the elastic locking tongue 22, which is conducive to mounting the liner 2 into the outer cup 1 more easily.

In embodiments of the present disclosure, the acetabular cup prosthesis may be further provided with an anti-rotation structure, i.e., as illustrated in FIGS. 1 to 2, a protrusion 26 is arranged on an outer edge of an opening of the liner 2, and a recessed portion 14 fitted with the protrusion 26 is arranged in an inner edge of an opening of the outer cup 1. The two portions cooperate with each other to prevent the rotation between the outer cup 1 and the liner 2. One or more protrusions 26 may be provided, and similarly one or more recessed portions 14 may be provided. A screw hole 15 may be arranged in a bottom of the outer cup 1 to facilitate fixing of the outer cup 1 into a human acetabulum through a screw; and/or, an instrument operation hole 16 may further be arranged in the bottom of the outer cup 1 to facilitate clamping of the outer cup 1 through an instrument. As illustrated in FIG. 1 (c), a connection line L3 is formed between any point on the abutting face 11 and a center of a circle of the opening portion 13 of the outer cup, and an angle β between the connection line L3 and a horizontal plane is greater than or equal to 45 degrees, preferably 45 degrees, so that a position of the abutting face 11 in the outer cup 1 is far away from the cup opening, so that the outer cup 1 may adapt to more models of the liner 2, and may be compatible with a polyethylene liner and a ceramic liner. In addition, the outer cup 1 is preferably made of metal, such as titanium alloy, and the liner 2 is preferably made of polyethylene.

In the description throughout the specification, "a plurality of' means at least two such as two or three, unless otherwise expressly and specifically defined.

In the present disclosure, unless otherwise expressly specified and defined, terms such as "mounting," "interconnection," "connection," "fixing" shall be understood broadly, and may be, for example, fixed connections, detachable connections, or integral connections; may also be mechanical or electrical connections or intercommunication; may also be direct connections or indirect connections via intervening media; may also be inner communications or interactions of two elements. For those skilled in the art, the specific meaning of the above terms in the present disclosure can be understood according to the specific situations.

Although embodiments of the present disclosure have been shown and described above, it can be understood that the above embodiments are exemplary and shall not be understood as limitation to the present disclosure, and changes, modifications, alternatives and variations can be made in the above embodiments within the scope of the present disclosure by those skilled in the art.

## Claims

1. An acetabular cup prosthesis, comprising an outer cup and a liner,
an inner surface of the outer cup being provided with an abutting face along a circumferential direction;
an outer surface of the liner being provided with a protruding ring at a bearing portion, and an elastic locking tongue at a non-bearing portion;
the protruding ring and the elastic locking tongue each abutting against the abutting face to self-lock the liner in the outer cup.

2. The acetabular cup prosthesis according to claim 1, wherein the inner surface of the outer cup is provided with an annular groove with a triangular longitudinal section along the circumferential direction, and a side wall of the annular groove close to an opening portion of the outer cup is the abutting face;
or, the inner surface of the outer cup is provided with an annular protrusion along the circumferential direction, and a side wall of the annular protrusion away from the opening portion of the outer cup is the abutting face.

3. The acetabular cup prosthesis according to claim 1, wherein the elastic locking tongue is formed by digging a tangential groove in the outer surface of the liner, and an orientation of the elastic locking tongue is facing away from a bottom of the liner.

4. The acetabular cup prosthesis according to any one of claims 1 to 3, wherein an extension line of a force of the abutting face on the elastic locking tongue is located below a midpoint at a root of the elastic locking tongue.

5. The acetabular cup prosthesis according to claim 4, wherein the elastic locking tongue has an inner side face, an outer side face parallel to the inner side face, and a top face perpendicular to the inner side face and the outer side face, an angle between the abutting face and the top face of the elastic locking tongue is 0-5 degrees.

6. The acetabular cup prosthesis according to claim 5, wherein an angle between the outer side face of the elastic locking tongue and a central axis of the liner is 40-50 degrees.

7. The acetabular cup prosthesis according to any one of claims 1 to 3, wherein an angle occupied by the protruding ring in the circumferential direction is 90-180 degrees.

8. The acetabular cup prosthesis according to claim 7, wherein the protruding ring is a continuous unitary section or a plurality of sections spaced apart from each other.

9. The acetabular cup prosthesis according to any one of claims 1 to 3, wherein an outer edge of an opening of the liner is provided with a protrusion, and an inner edge of an opening of the outer cup is provided with a recessed portion fitted with the protrusion.

10. The acetabular cup prosthesis according to any one of claims 1 to 3, wherein a connection line is formed between any point on the abutting face and a center of a circle of the opening portion of the outer cup, and an angle between the connection line and a horizontal plane is greater than or equal to 45 degrees.

11. A locking structure for an acetabular cup prosthesis, the acetabular cup prosthesis comprising an outer cup and a liner, the liner being locked in the outer cup through the locking structure, wherein
an inner surface of the outer cup is provided with an abutting face along a circumferential direction;
an outer surface of the liner is provided with a protruding ring at a bearing portion, and an elastic locking tongue at a non-bearing portion;
the protruding ring and the elastic locking tongue each abut against the abutting face to self-lock the liner in the outer cup.

12. The locking structure according to claim 11, wherein the inner surface of the outer cup is provided with an annular groove with a triangular longitudinal section along the circumferential direction, and a side wall of the annular groove close to an opening portion of the outer cup is the abutting face;
or, the inner surface of the outer cup is provided with an annular protrusion along the circumferential direction, and a side wall of the annular protrusion away from the opening portion of the outer cup is the abutting face.

13. The locking structure according to claim 11, wherein the elastic locking tongue is formed by digging a tangential groove in the outer surface of the liner, and an orientation of the elastic locking tongue is facing away from a bottom of the liner.

14. The locking structure according to claim 11, wherein an extension line of a force of the abutting face on the elastic locking tongue is located below a midpoint at a root of the elastic locking tongue.

15. The locking structure according to claim 14, wherein the elastic locking tongue has an inner side face, an outer side face parallel to the inner side face, and a top face perpendicular to the inner side face and the outer side face, an angle between the abutting face and the top face of the elastic locking tongue is 0-5 degrees.

16. The locking structure according to claim 15, wherein an angle between the outer side face of the elastic locking tongue and a central axis of the liner is 40-50 degrees.

17. The locking structure is according to claim 11, wherein an angle occupied by the protruding ring in the circumferential direction is 90-180 degrees.

18. The locking structure according to claim 17, wherein the protruding ring is a continuous unitary section or a plurality of sections spaced apart from each other.

19. The locking structure according to claim 11, wherein an outer edge of an opening of the liner is provided with a protrusion, and an inner edge of an opening of the outer cup is provided with a recessed portion fitted with the protrusion.

20. The locking structure according to claim 11, wherein a connection line is formed between any point on the abutting face and a center of a circle of the opening portion of the outer cup, and an angle between the connection line and a horizontal plane is greater than or equal to 45 degrees.
